(19) Europäisches Patentamt
European Patent Office
Office européen des brevets

(11) Veröffentlichungsnummer : **0 305 389 B1**

# (12) EUROPÄISCHE PATENTSCHRIFT

(45) Veröffentlichungstag der Patentschrift : **22.01.92 Patentblatt 92/04**

(51) Int. Cl.$^5$ : **C07C 331/00,** C07D 317/72, C07F 7/18

(21) Anmeldenummer : **87903245.6**

(22) Anmeldetag : **18.05.87**

(86) Internationale Anmeldenummer : **PCT/DE87/00231**

(87) Internationale Veröffentlichungsnummer : **WO 87/06932 19.11.87 Gazette 87/25**

## (54) VERFAHREN ZUR SYNTHESE HOMOCHIRALER FÜNF- UND SECHSRING- ZWISCHENPRODUKTE.

(30) Priorität : **16.05.86 DE 3616850**

(43) Veröffentlichungstag der Anmeldung : **08.03.89 Patentblatt 89/10**

(45) Bekanntmachung des Hinweises auf die Patenterteilung : **22.01.92 Patentblatt 92/04**

(84) Benannte Vertragsstaaten : **AT BE CH DE FR GB IT LI LU NL SE**

(56) Entgegenhaltungen : **THE JOURNAL OF ORGANIC CHEMISTRY, Band 45, Nr. 2, 18. Januar 1980, American Chemical Society, US; C. R. JOHNSON et al, "S-ethenylsulfoximine derivatives. Reagents for ethylenation of protic nucleophiles", Seiten 264-271, siehe S. 264**

(56) Entgegenhaltungen : **JOURNAL OF THE AMERICAN CHEMICAL SOCIETY, Band 104, Nr. 14, 14. Juli 1982, American Chemical Society, US; C. R. JOHNSON et al, "Sulfoximine-mediated resolutions of ketones", S. 4021-4023, siehe S. 4022**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **GAIS, Hans-Joachim**
**In den Weihermatten 28**
**W-7800 Freiburg/Breisgau (DE)**
Erfinder : **ERDELMEIER, Irene**
**Falterweg 22**
**W-7800 Freiburg/Breisgau (DE)**
Erfinder : **BIRK, Rolf**
**Seitzstr. 4**
**W-7800 Freiburg/Breisgau (DE)**

EP 0 305 389 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur E/Z-stereoselektiven Synthese homochiraler 5- und 6-Ring-Zwischenprodukte der Formel I

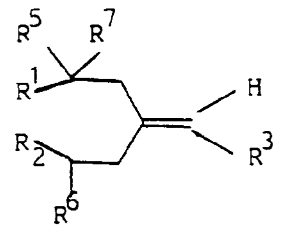

(I),

worin R 1 und R 2 eine gemeinsame Bindung oder den Rest

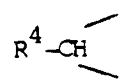

,

R 3 die Gruppe

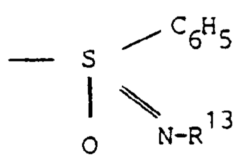

$R^4$ einen geradkettigen oder verzweigten Alkylrest mit 1-10 C-Atomen $R^5$ und $R^6$ gleich oder verschieden sind und Wasserstoff, Alkyl mit 1-10 C-Atomen, Cycloalkyl mit 5-7 C-Atomen, Alkoxy mit 1-6 C-Atomen, Aryl mit 6-10 C-Atomen, Aralkyl mit 7-12 C-Atomen oder einen 5-7-gliedrigen Heterocyclus, der ein weiteres N-, O- oder S-Atom enthalten kann, oder wenn $R^1$ und $R^2$ eine gemeinsame Bindung darstellen $R^5$ und $R^6$ gemeinsam die

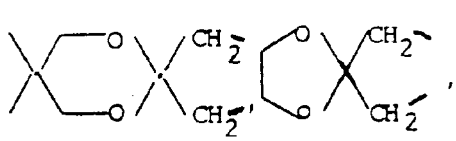

,

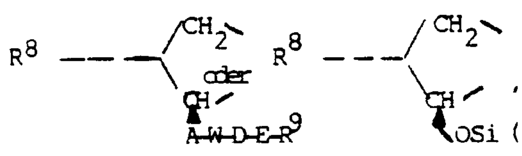

oder

, wobei

$R^8$ Wasserstoff, Alkyl mit 1-10 C-Atomen oder $OR^{10}$ mit $R^{10}$ in der Bedeutung eines Wasserstoffs, eines Silylrestes oder eines Äther- oder Säurerestes darstellt,
A eine trans-CH=CH-Gruppe oder -C≡C-Gruppe,
W eine Hydroxymethylen- oder- $-C(CH_3)(OH)$-Gruppe,
D eine Alkylengruppe mit 1-5 C-Atomen,
E eine -C≡C- oder $-CH = CR^{11}$-Gruppe,
$R^9$ eine Alkylgruppe mit 1-6 C-Atomen,
$DER^9$ Cycloalkyl mit 5-6 C-Atomen oder den Rest

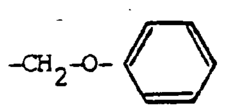

,

$R^{11}$ Alkyl mit 1-4 C-Atomen,
$R^7$ die Reste - $(CH_2)_m-R^{12}$, oder

$$-(CH_2)_{m-o}-\left[Z_1-(CH_2)_{m-p}\right]_x-\left[Z_2-(CH_2)_{m-q}\right]_y-R^{12},$$

Wasserstoff
$R^{13}$ Alkyl mit 1-4 C-Atomen oder einen Tosylrest,
$R^{14}$ Alkyl mit 1-4 C-Atomen, Phenyl oder Benzyl,
m = 2-20,
o, p und q≦16,
x, y = 0,1 oder 2,
$Z_1$ eine cis-CH=CH-Gruppe, eine trans-CH=CH-Gruppe oder eine -C≡C-Gruppe darstellt,
$Z_2$ Sauerstoff, Schwefel, eine NH-, eine N-Methylgruppe oder eine -C≡C-Gruppe bedeutet und
$R^{12}$ freies oder geschütztes Amino, Methylamino, Hydroxy, Carboxy, Mercapto oder Halogen bedeuten, das dadurch gekennzeichnet ist, daß man an prochirale symmetrische Ketone der Formel II.

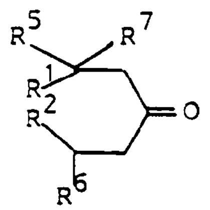
(II),

worin $R^1$, $R^2$, $R^5$, $R^6$ und $R^7$ die oben angegebenen Bedeutungen haben, das Lithiosulfoximin aus n-Butyllithium und einem N-substituierten S-Methyl-S-phenyl-sulfoximin-Derivat der Formel III

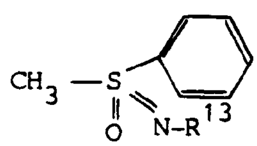
(III),

worin $R^{13}$ die bereits angegebene Bedeutung hat, anlagert und anschließend mit n-Butyllithium, Trimethylchlorsilan umsetzt.

Die Umwandlung von symmetrischen Ketonen in unsymmetrische, chirale Olefine stellt weitgehend noch ein ungelöstes Problem dar. Bisher gelang es nur in einzelnen Fällen unter Verwendung von speziell dafür hergestellten Reagenzien derartige asymmetrische Wittig-ähnliche Reaktionen durchzuführen [s.S. Hanessian et al. JACS 106 (1984), 5754]. Ein in J. Org. Chem. 45 (1980), 264 beschriebener Synthese weg führt zu unterschiedlichen Ergebnissen und eignet sich nur in bestimmten Fällen zur Synthese chiraler Olefine.

L.R. Johnson und J.R. Zeller setzen Lithiosulfoximine mit Ketonen zur Trennung von Diastereomeren um (J. Am. Chem. Soc. 104 (1982), 4021).

Es wurde nun überraschend gefunden, daß sich insbesondere Lithiosulfoximine aus n-Butyllithium und den nach C.R. Johnson et al. [JACS 95 (1973), 7418] leicht herstellbaren chiralen N-substituierten S- M ethyl-S-phenylsulfoximin-Derivaten an prochirale symmetrische Ketone anlagern un anschließend mit einer Alkyl- oder Aryllithiumverbindung, vorzugsweise n-Butyllithium/Trimethylchlorsilan zu den auf andere Weise nur schwer herstellbaren unsymmetrischen Olefinen der Formel I umgesetzt werden können.

Für die Alkylreste $R^4$, $R^5$, $R^6$ und $R^8$ kommen alle geradkettigen und verzweigten Reste mit 1-10 C-Atomen in Betracht, wie z.B. Methyl, Ethyl, Propyl, Isopropyl, Butyl, sek.-Butyl, tert.-Butyl, Isobutyl, Pentyl, Isopentyl, Neopentyl, Hexyl, Isohexyl, Heptyl, Octyl, Nonyl, Decyl. Bevorzugte Reste sind die mit 1-6 C-Atomen.

Als Cycloalkyl mit 5-6 C-Atomen für DE$R^9$, $R^5$ und $R^6$ sind Cyclopentyl und Cyclohexyl und die jeweiligen Methyl-substituierten Derivate gemeint.

Die $C_{1-6}$-Alkylgruppen $R^9$, die $C_{1-4}$-Alkylgrupen $R^{11}$, $R^{13}$ und $R^{14}$ sowie die $C_{1-6}$-Alkoxygruppen $R^5$ und $R^6$ entsprechen ebenfalls den für $R^4$ bereits gennanten geradkettigen und verzweigten Alkylresten.

Die Arylreste mit 6-10 C-Atomen ($R^5$ und $R^6$) sollen in erster Linie Phenyl, α- und β-Naphthyl darstellen. Die Aralkylreste $R_5$ und $R_6$ mit 7-12 C-Atomen sind Benzyl, Phenethyl, 2-Phenethyl, 3-Phenylpropyl, α-Naphthylmethyl.

Die wichtigsten 5-7-gliedrigen Heterocyclen, die noch ein weiteres N-, O- oder S-Atom enthalten können, sind

Pyrryl, Furyl, Thienyl, Pyrazolyl, Imidazolyl, Thiazolyl, Pyridyl, Pyrymidinyl, Pyrazinyl, Azepinyl, Diazepinyl.

Die Hydroxygruppen in W können funktionell abgewandelt sein, beispielsweise durch Verätherung oder Veresterung, wobei die freien oder abgewandelten Hydroxygruppen in W $\alpha$- oder $\beta$-ständig sein können, wobei freie Hydroxygruppen bevorzugt sind.

Als Äther- und Acylreste (z.B. auch für $R^{10}$) kommen die dem Fachmann bekannten Reste in Betracht. Bevorzugt sind leicht abspaltbare Ätherreste wie beispielsweise der Tetrahydropyranyl-, Tetrahydrofuranyl-, $\alpha$-Äthoxyäthyl-, Trimethylsilyl-, Dimethyl-tert.-butyl-silyl-, Tri- benzyl-silyl- und Diphenyl-tert.-butylsilylrest. Als Acylreste kommen in Frage: Acetyl, Propionyl, Butyryl, Benzoyl.

Als Alkylengruppe D kommen geradkettige oder verzweigtkettige, gesättigte Alkylenreste mit bis zu 5 C-Atomen in Frage, die gegebenfalls durch Fluoratome, 1,2-Methylen, 1,1-Trimethylen, 1,1-Tetramethylen oder 1,1-Pentamethylen substituiert sein können. Beispielsweise seien genannt: Methylen, Fluormethylen, Äthylen, Methyläthylen, Äthyläthylen, Trimethylen, Tetramethylen, Pentamethylen, 1-Methyltetramethylen, 1-Methyl-trimethylen, Pentamethylen, 1-Methyltetramethylen, 1-Methyl-trimethylen, 1,1-Trimethylenäthylen, 1,1-Tetramethylenäthylen.

Für $R^7$ als $-(CH_2)_m-R^{12}$ kommen Alkylengruppen mit 2-20 C-Atomen in Betracht, die noch eine oder mehrere Gruppen $Z_1$ oder $Z_2$ enthalthen können wie $-(CH_2)_{m-o}-[Z_1-(CH_2)_{m-p}]_x-[Z_2-(CH_2)_{m-q}]_y-R^{12}$ wobei m = 2-20 und o, p und q zusammen $\leqq$ 16 ist, wie z.B. $-(CH_2)_5-NH_2$, $-(CH_2)_6-NHCH_3$, $-(CH_2)_2-0-(CH_2)_2-COOH$, $-(CH_2)_2-0-(CH_2)_3-NH_2$, $-(CH_2)_2-0-(CH_2)_2-0-(CH_2)_2-OH$,

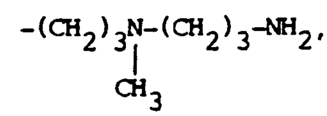

$-CH_2-C\equiv C-(CH_2)_2-NH_2$, $-(CH_2)_2-C\equiv C-(CH_2)_2-NH_2$, $-(CH_2)_2-C\equiv C-(CH_2)_2-0-(CH_2)_2-SH$, $-C\equiv C-(CH_2)_{4-5}-NH_2$, $-C\equiv C-(CH_2)_3-C\equiv C-(CH_2)_2-NH_2$.

Nach dem erfindungsgemäßen Verfahren lassen sich die homochiralen Olefine der Formel I in hohen Ausbeuten darstellen, wobei sie praktisch in optisch reiner Form anfallen. Damit erweist sich das neue Verfahren insbesondere bei enolisierbaren 5-Rtngketonen der in Tetr. Letters 26, 4359 (1985) beschriebenen Methode weit überlegen. Besonders interessante Zwischenprodukte stellen Olefine mit einem Bicyclo [3.3.0] octan-Ring-Gerüst dar, da sie zu den biologisch aktiven (E)-Carbacyclinen, wie z.B. Iloprost, führen.

Um zu diesen biologisch interessanten Verbindungen zu gelangen, muß die Sulfoximinogruppe $R^3$ in den Olefinen der Formel I durch einen substituierten Alkylrest, insbesondere durch eine $C_4$-Einheit [vgl. hierzu E.J. Corey et al., Tetr. Letters 24, 5571 (1983)], mit Hilfe von metallorganischen Methoden wie z.B. der von m. Julia et al., Tetr. Letters 23, 2469 (1982), substituiert werden.

Die Erfindung betrifft die Addition von chiralen (R)- oder-(S)-Phenylsulfoximinmethylenlithiumverbindungen an prochirale Ketone der Formel II, vorzugsweise Cyclopentanon- oder Cyclohexanonverbindungen, und die anschließende Umsetzung mit einer aktivierten Trialkylsiliziumverbindung unter Abspaltung von Silanol-Lithiumsalzen zu chiralen Olefinen der Formel I entsprechend Anspruch.

Unter geschützter Aminogruppe $R^{12}$ sind zu verstehen: Acyl wie z.B. Acetyl, Propionyl oder Benzoyl, Urethanschutzgruppen wie z.B. Benzyloxycarbonyl, tert.-Butyloxycarbonyl oder 9-Fluorenylmethyloxycarbonyl und Phthalimidoyl. Dies soll nur eine Auswahl von möglichen Schutzgruppen darstellen. Unter Halogen als $R^{12}$ sind gemeint Fluor, Chlor und Brom.

Anstelle des n-Butyllithium in der Kombination n-Butyllithium/Trimethylchlorsilan eignen sich auch noch andere Alkyl- bzw. Aryllithium-Derivate wie z.B. $CH_3Li$, $C_2H_5$-Li, $C_3H_7Li$, tert.-Butyl-Li, sek.-Butyl-Li oder Phenyl-Li.

Die Reaktionsbedingungen in den Arbeitsvorschriften A 1 und A 2 stellen nur die bevorzugten Bedingungen dar.

Die vorliegende Erfindung umfaßt natürlich auch alle vom Durchschnittsfachmann herleitbaren Abwandlungen.

Beispiele:

Arbeitsvorschrift A 1 zur Darstellung der 2-Hydroxysulfoximine 2, 5, 10 und 12.

Zu einer Lösung von N, S-Dimethyl-S-phenyl-sulfoximin (1) in absolutem THF wird unter Stickstoff bei 0 °C 1 Äquivalent einer 1,5-normalen Lösung von n-Butyllithium in n-Hexan tropfenweise so zugegeben, daß die Temperatur der Lösung nicht über 5 °C steigt. Nach vollendeter Zugabe wird noch 15 Min. bei 0 °C gerührt und anschließend die leicht gelbliche Lösung des Lithiosulfoximins auf -78 °C gekühlt. Ein Äquivalent der entsprechenden Carbonylverbindung, gelöst in einigen ml absolutem THF, wird dann auf einmal mit der Spritze zugegeben, wodurch die Temperatur der Lösung um 5-20 Grad ansteigt. Nach Abklingen der exothermen Reaktion wird das Kühlmedium entfernt und man läßt auf Raumtemperatur erwärmen. 1 h nach Zugabe der Carbonylverbindung wird das Reaktionsgemisch mit gesättigter $NH_4Cl$-Lösung gequencht und mit Essigester extrahiert, wobei im allgemeinen eine Extraktion genügt (DC-Kontrolle; sehr empfindliche Anfärbereaktion mit Anisaldehyd-Eisessig-Sprühreagenz). Nach Trocknen mit $MgSO_4$, Einengen im Vakuum und FC an Kieselgel (Hexan/Essigester-Gemisch) erhält man die Produkte als farblos-kristalline Feststoffe. Da die $R_f$-Werte der Addukte zwischen dem der Ausgangsverbindung 1 und der jeweiligen Carbonylverbindung liegen, kann bei der FC gleichzeitig unumgesetzte Carbonylverbindung zurückgewonnen werden.

Arbeitsvorschrift A 2 zur Darstellung der 1-Alkenylsulfoximine 3, 6, 8, 11 und 13.

Zu einem Aquivalent des Additionsproduktes nach A 1 in absolutem THF wird unter $N_2$ bei 0 °C genau ein Äquivalent einer 1,5-normalen Lösung von n-Butyllithium in n-Hexan zugegeben, wobei bei Zugabe des letzten Tropfens die Reaktionslösung schlagartig eine gelbe Farbe annimmt. Man rührt 15 Min. bei 0 °C, kühlt dann auf -78 °C, gibt auf einmal 1.3 Äquivalente Trimethylchlorsilan (pur oder als Lösung in absoluten Hexan) zu und läßt auf Raumtemperatur erwärmen. Nach 3 h bei Raumtemperatur kühlt man die inzwischen wieder farblose Lösung auf -78 °C ab und gibt tropfenweise (unter Orangefärbung der Lösung) 1 Äquivalent der n-Butyllithiumlösung zu und rührt bei -70 bis -90 °C. Nach Beendigung der Reaktion nach 0.5.-2.5 h (DC: Die Produkte haben grundsätzlich einen kleineren $R_f$-Wert als die Edukte) wird mit ges. $NH_4Cl$-Lösung gequencht und mit Essigester extrahiert. Nach dem Trocknen mit $MgSO_4$ und Einengen erhält man nach FC an Kieselgel mit Hexan/Essigester-Gemischen das Produkt; nicht vollständig umgesetztes Edukt kann bei der FC wieder zurückgewonnen werden.

1.) (±)-1-[(N-methyl-S-phenylsulfonimidoyl]-cyclopentanol (2).

Nach A 1 werden 3.38 g (20 mmol) (±) -N, S-Dimethyl-S-phenyl-sulfoximin 1 in 50 ml absoluten THF mit 13.3 ml (20 mmol) einer Butyllithium-n-Hexan-Lösung metalliert und anschließend mit 1.68 g (20 mmol) Cyclopentanon umgesetzt. Nach FC an 130 g Kieselgel (n-Hexan/Essigester 1:1) erhält man 4.7 g (93 %) (±) -2 als farblos-kristalline Verbindung, Schmp. 67-68 °C (Hexan/Essigester).

| | | | | | |
|---|---|---|---|---|---|
| $C_{13}H_{19}NO_2S$ (253.4) | Ber. | C 61.63 | H 7.56 | N 5.53 |
| | Gef. | C 61.34 | H 7.65 | N 5.48 |

1H-NMR (300 MHz, $CDCl_3$): δ = 1.33-2-23 (m, 8H. 2-H, 4-H, 5-H), 2.61 (s, 3H, $N-CH_3$), 3.10 (d, 1H, J = 14 Hz, 6-H), 3.57 (d, 1H, J = 14 Hz, 6-H), 6.24 (s, breit, 1H, -OH), 7.52-7.67 (m, 3H, Ar-H), 7.83-7.91 (m, 2H, Ar-H). MS (EI, 70 eV): m/z (%) = 253 ($M^+$,4), 211 (18), 156 (80), 140 (59), 125 (PhSO, 100), 107 (66), 106 (32), 91 (28), 81 (40), 78 (48), 77 (40), 51 (28), 41 (32).

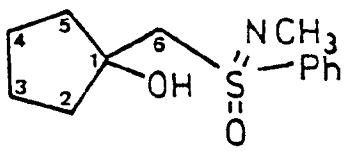

2

2.) (±)-S-Cyclopentylidenmethyl-N-methyl-S-phenylsulfoximin (3).

760 mg (3 mmol) (±)-2 in 30 ml absoluten THF werden nach A 2 umgesetzt, wobei die Eliminierung schon 15 min. nach Zugabe des zweiten Äquivalents n-Butyllithium vollständig ist. Nach Säulenfiltration über Kieselgel (Hexan/Essigester 1:1) erhält man 700 mg (99 %) farblos-kristallines (±)-3, Schmp. 81-82 °C (Essigester).

| | | | | | |
|---|---|---|---|---|---|
| $C_{13}H_{17}NOS$ (235.3) | Ber. | C 66.35 | H 7.28 | N 5.95 |
| | Gef. | C 66.22 | H 7.22 | N 6.09 |

$^1$H-NMR (300 MHz, $CDC1_3$): δ = 1.52-1.80 (m, 4H, 3-H, 4-H), 2.26-2.50 (m, 3H, 2-H, 5-H), 2.67 (s, 3H, N-$CH_3$), 2.78-2.94 (m, 1H, 2-H), 6.31 (quint, 1H, J = 2.5 Hz, 6-H), 7.48-7.62 (m, 3H, Ar-H), 7.86-7.94 (m, 2H, Ar-H).
MS (EI, 70 eV): m/z (%) = 236 ($M^+$+1, 15), 235 )$M^+$, 77), 234 ($M^+$-1, 16), 207 (12), 205 (19), 189 (15), 187 (42), 157 (36), 156 (50), 155 (100), 129 (51), 126 (12), 125 (22), 115 (17), 110 (15), 109 (33), 107 (13), 106 (14), 105 (12), 97 (14), 91 (30), 81 (49), 79 (75), 78 (30), 77 (63), 67 (30), 65 (18), 53 (40), 51 (44), 50 (12), 46 (12), 43 (61), 52 (65), 40 (41).

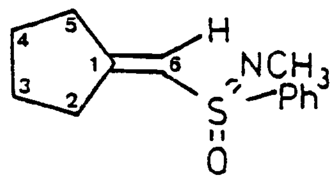

3

3.) (±)-5′-[[(N-Methyl-S-phenyl)-sulfonimidoyl]-methyl]-tetrahydrospiro-[1.3-dioxolan-2,2′-(3′aα, 5′β, 6′aα)-1H, 3′H-pentalen -5′ol [(±)5] und [(+)- 5′ (S)]-5].

a) (±)-5

1.69 g (10 mmol) (±)-1 werden A 1 in 50 ml absoluten THF metalliert und mit 1.82 g (10 mmol) [Spiro- 1′,3′-dioxolan-2,2′-tetrahydro (1H)-pentalen]-5 (3H)-on (4) bei -78 °C umgesetzt. Nach 15 Min. bei - 78 °C ist die Reaktion nahezu vollständig. FC des Rohprodukts an 130 g Kieselgel (Hexan/Essigester 1:2) liefert 3.4 g (96 %) (±)-5 in farblosen langen Nadeln, Schmp. 105-108 °C.

$C_{18}H_{25}NO_4S$ (351.5) Ber. C 61.50 H 7.17 N 3.98
Gef. C 61.37 H 7.22 N 4.01

b) (+)- 5′(S) -5

2.03 g (12 mmol) S-1 (ee≧96 %) werden wie in a) mit 2.18 g (12 mmol) 4 umgesetzt. Man erhält 3.88 g (92 %) (+) -[5′(S)]-5 in langen Nadeln, Schmp. 132-133 °C, $[\alpha]_D^{20}$= + 36.4°, $[\alpha]_{546}^{20}$ = +43.5° (c = 1.6 in Aceton). Bei Vergrößerung des Ansatzes können 75 % des Produktes durch einmaliges Umkristallisieren des Rohgemisches (Hexan/Essigester) rein erhalten werden, den Rest erhält man durch anschließende FC der eigeengten Mutterlauge.

Ber. C 61.50 H 7.17 N 3.98
Gef. C 61.50 H 7.22 N 3.98

$^1$H-NMR (300 MHz, $CDC1_3$): δ = 1.70 (dd, 1H, $J_{1'a,1'\beta}$= 13 Hz, $J_{a'\beta,6'aa}$= 9Hz, 1′Ha (oder 3′-Ha), 1.83 (dd, 1H, $J_{1'\beta,1'a}$= 13 Hz, $J_{1'\beta,6'aa}$= 6 Hz, 1′-Hβ (oder 3′-Hβ), 1.88-2.10 (m, 5H, 2x1′-H) (oder 2x3′-H), 2 x 6′-H, 4′Hβ), 2.42-2.55 (m, 2H, 3a′-Ha, 6a′-Ha), 2.63 (s, 3H,N-$CH_3$), 2.60-2.74 (ddd, 1H, $J_{4'a,4'\beta}$ =15 Hz, $J_{4'a/3'aa}$ =8Hz, $J_{4'a/6'a}$ =2 Hz. 4′-Ha), 3.06 (d, 1H, J = 14 Hz, 7′-H), 3.46 (d, 1H, J = 14 Hz, 7′-H), 3.90 (m, 4H, 4-H, 5-H), 6.43 (s, breit, 1H, -OH), 7.55-7.70 (m, 3H, Ar-H), 7.84-7.93 (m, 2H, Ar-H).
MS (EI, 70 eV): m/z (%) = 351 ($M^+$,13), 196 (17), 182(µ1), 169(14), 156 (100), 154 (PhSONC$H_3$, 37), 140 (36), 139 (PhSON, 24), 135 (14), 125 (PhSO, 85), 113 (38), 112(32), 107 (40), 106(30), 99(21), 95(27), 91 (19), 86 (24), 79(11), 78(19), 77(39), 55(13), 51(20), 43(25), 41(48), 39(17).

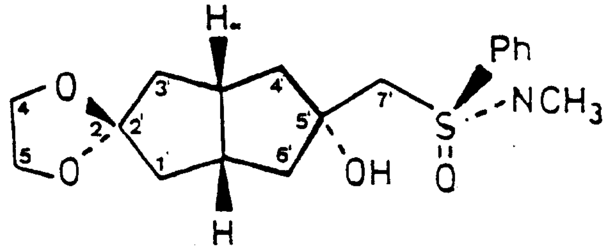

(+)-5'α(S)-5

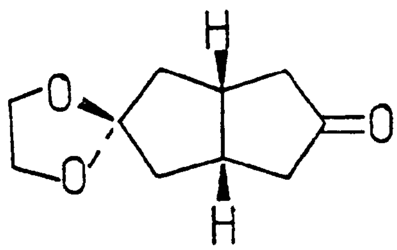

4

4.) (±)-S-Spiro-[1,3-dioxolan-2,2'-[(3'aα,6'aα)-tetrahydro(1H,3H)-5-pentalenoyliden]] methyl-N-methyl-S-phenyl-sulfoximin [(-)-[(aR)-S-(S)]-6] und [(±)-6]

a) (±)-6

525 mg (1.5 mmol) (±)-5 werden in 30 ml absoluten THF nach A 2 umgesetzt. 90 Min. nach Zugabe des zweiten Äquivalents der n-Butyllithiumlösung wird aufgearbeitet. FC an 100 g Kieselgel (Essigester) liefert 450 mg (90 %) eines leicht gelblichen Öls, das aus diasteromerenreinem (±)-6 (de ≧ 96 %, laut $^1$H-NMR bei 300 MHz) neben 11 % isomeiisiertem (±)-6i besteht.

b) (-)-[(aR)-S(S)]-6

525 mg (1.5 mmol) (+)-[5' (S)]-5 werden wie in a) umgesetzt. Das Produkt (-)-[(aR)-S(S)]-6, $[\alpha]_D^{20}$ = -64.9° (c = 1.5 in $CH_2C1_2$) enthält 14 % der isomerisierten Verbindung 6i.

$C_{18}H_{23}NO_3S$ (333.1) Ber. C 64.83 H 6.95 N 4.20
(333.1412, Gef. C 64.43 H 6.96 N 4.06
333.14134 Hochauflösung)

$^1$H-NMR (300 MHz, $CDC1_3$) Gemisch aus 6 und 6 i

6: = 1.56-1.70 (2 x dd, 2H, $J_{1'a/1'ß} = J_{3'a/3'ß}$ = 15Hz, $J_{3'/3'a} = J_{1'/6'aa}$ = 8 Hz, 1-H,3-H), 1.96-2.10 (2 x dd, J = 15 Hz, J = 8 Hz, 1-H, 3-H), 2,23-3,17 (m, 6H, 4-H, 6-H, 3a-Ha, 6a-Ha), 2.68 (s, 3H, N-$CH_3$), 3.86 (s, 4H, 4-H, 5-H), 6.27 (quint, 1H, J = 2 Hz, 7'-H), 7.48-6.63 (m, 3H, Ar-H), 7.80-7.94 (m, 2H, Ar-H).

Für 6i werden nur die Protonen mit gegenüber 6 unterschiedlicher chemischer Verrschiebung angegeben. 6i: δ = 1.23-1.38 (m, 1′-H/3′-H), 1.43-1.56 (m, 1′-H/3′-H),1.85-1.96 (m. 1′-H/3′-H), 2.71 (s, N-$CH_3$), 2.72 (s, N-$CH_3$), 3.87 (s, 4-H, 5-H), 3.95 (s, breit, 7-H), 5.21 (m, 4-H), 5.27 (m, 4-H).

$^{13}$C-NMR (75.47 MHz, $CDCl_3$):

6: δ = 29.22 (q, N-$CH_3$), 35.86 (t), 39.20, 41.19 (2 x d, 3′a-C, 6′a-C), 41.66, 41.69, 42.05 (3x t, 1′-C, 3′-C, 4′-C, 6′-C), 63.94, 64.53 (2 x t, 4-C, 5-C), 118.46 (s, 2-C), 122,42 (d, 7′-C), 128.53, 129.01 (2 x d, Ar-C), 132.12 (d, Ar-C), 140.97 (s, Ar-C), 163.72 (s, 7′-C).
61i: δ = 30.0, 38.67, 39.84, 41.54, 41.89, 42.17, 47.51, 58.35, 64.71, 117, 129.62, 129.69, 132.73, 138.49.
MS (EI, 70 eV): m/z (%) = 333 ($M^+$, 100), 246(55), 208 ($M^+$-PhSO, 32), 179 ($M^+$-$PhSONCH_3$, 38).

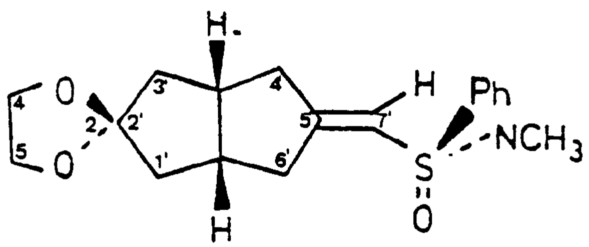

(-)-[(aR)-S(S)]-6

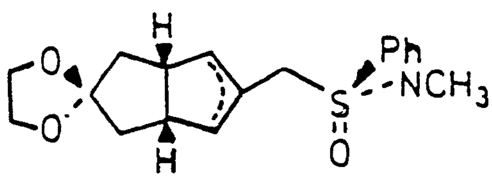

6i

5.) (±)-cis/trans-4- (1,1-Dimethyl)-1-[(N-methyl-S-phenylsulfonimidoyl)-methyl]-cyclohexanol (±)-7a/(±)-7b und (+)-7a/(+)-7b

a) (±)-7b, (±)-7a

Nach C.R. Johnson, C.N. Schroeck und J.R. Shanklin, JACS 95, 7424 (1973) ergibt die Addition von (±)-1 an t-Butylcyclohexanon ein kristallines Gemisch aus (±)-7a und (±)-7b. Durch Kristallisation aus n-Hexan erhält man reines (±)-7a neben einem an (±)-7b angereichertem Gemisch (±)-7b:(±)-7a= 5.4:1 (laut $^1$H-NMR, 300 MHz).

b) (+)-7a/(+)-7b

Analog zu a) wird S-1 mit t-Butylcyclohexanon umgesetzt und das gereinigte Gemisch (+)-7, das zu 29 % aus 7a und 71 % aus 7b besteht (aus $^1$H-NMR, 300 MHz) ohne weitere Kristallisation in beispiel 6c eingesetzt.

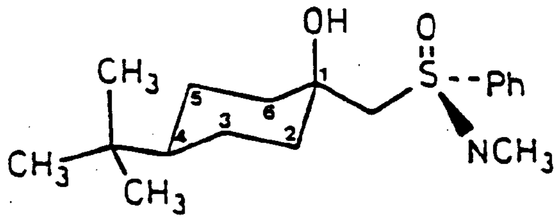

7a

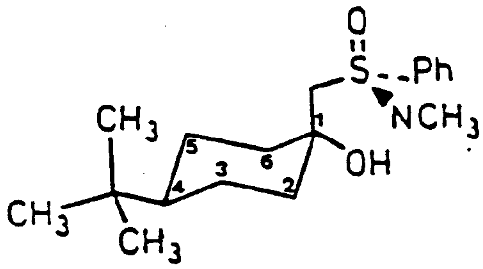

7b

6.) (±)-S-[[4-(1,1-Dimethylethyl)-cyclohexyliden]-methyl]-N-methyl-S-phenyl-sulfoximin (±)-8a, (±)-8b und (+)-(aS)-8a und (-)-(aR)-8b

a) Umsetzung von (±)-7a/(±)-7b

965 mg (3mmol) des nach 5a) dargestellten Gemisches aus (l)-7a und (±)-7b (1:5.4) werden in 50 ml absoluten THF nach A 2 umgesetzt. 2 h nach Zugabe des 2. Äquivalents n-Butyllithium-Lösung wird wie beschrieben aufgearbeitet; nach FC an 100 g Kieselgel (Essigester/Hexan 2:1) erhält man 850 mg (93 % eines farblosen Öls, das im Kühlschrank erstarrt. Laut $^1$H-NMR (300 MHz) besteht das Produkt aus den Diastereomeren (±)-8a und (±)-8b im Verhältnis 87:13. Dünnschichtchromatographisch sind beide Diastereo- mere getrennt zu erkennen.

| | | | | |
|---|---|---|---|---|
| $C_{18}H_{27}NOS$ (305.5) | Ber. | C 70.77 | H 8.91 | N 4.58 |
| | Gef. | C 70.81 | H 8.91 | N 4.58 |

b) Umsetzung von (±)-7a

483 mg (1.5 mmol) (±)-7a werden in 30 ml absoluten THF wie in a) umgesetzt. FC des Rohgemisches an 100 g Kieselgel (Hexan/Essigester 1:1) liefert neben 110 mg (23 %) unumgesetztem (±)-7a (aus $^1$ H-NMR) 330 mg (72 %, 94 % umsatzbezogen) der Diastereomeren (±)-8a und (±)-8b im Verhältnis 31:69 (aus $^1$ H-NMR, 300 MHz).

Da offensichtlich die Selektivität der Eliminierung aus 7a umgekehrt wie aus 7b ist, kann anhand der Versuche 6a und 6b auch die Selektivität der Eliminierung an reinem 7b berechnet werden.

| | | (±)-8a:(±)-8b |
|---|---|---|
| Eliminierung an | (±)-7a: | 31:69 |
| " | (±)-7a/(±)-7b (1:5.4): | 87:13 |
| " | 7b: | 98:2 |

c) Umsetzung von (+)-7a/7b

970 mg (3mmol) (+)-7a/7b (29 % 7a, 71 % 7b) werden in 30 ml absoluten THF nach A 2 umgesetzt. 1 h nach Zugabe des 2. Äquivalents n-Butyllithium wird aufgearbeitet, man erhält nach FC an 130 g Kieselgel (Essigester/Hexan 1:1) 865 mg (95 %) der Diastereomeren (+)-(aS)-8a und (-)-(aR)-8b im Verhältnis 78:22 (aus $^1$H-NMR, 300 MHz). Durch FC an feinem Kieselgel können beide Diastereomere isoliert werden. (+)-(aS)-8a in

farblosen langen Nadeln vom Schmp. 72° (Hexan) $[\alpha]_D^{20}$ = +46°, $[\alpha]_{546}^{20}$ = +51° (c = 0.5 in Aceton) und (-)-(aS)-8b als schwach gelbliches Öl: $[\alpha]_{20}^{D}$ = -99°, $[\alpha]^{20}_{546}$ = 123° (c = 0.25 in Aceton).

(+)-(aS)-8a

$C_{18}H_{27}NOS$ (305.5) Ber. C 70.77 H 8.91 N 4.58
Gef. C 70.66 H 8.97 N 4.50

Durch RSA eines aus Hexan gezüchteten Einkristalls von (+)-(aS)-8a konnte die Konfiguration bzgl. der chiralen Achse im Molekül als "S" festgelegt werden.

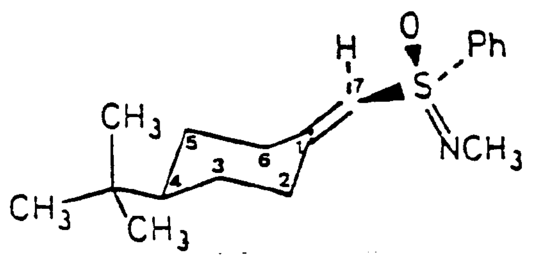

(+)-(aS)-8a

<u>$^1$H-NMR</u> (300 MHz, $CDCl_3$): δ = 0.56 (dq, 1H, $J_{3a/2a}$ = 14 Hz, $J_{3a/3e}$ = 13 Hz, $J_{3a/4a}$ = 12.5 Hz, $J_{3a/2e}$ = 2.5 Hz, 3-Ha), 0.75 (s, 9H, tert-Butyl-H), 1.03 (dq, 1H, $J_{5a/4a}$ = 13 Hz, $J_{5a/6a}$ = 12.5 Hz, $J_{5a/5e}$ = 12 Hz, $J_{5a/6e}$ = 4 Hz, 5-Ha), 1.15 (tt, 1H, $J_{4a/5a}$ = 13 Hz, $J_{4a/3a}$ = 12.5 Hz, $J_{4a/5e}$ = 3 Hz, 4-Ha), 1.72 (dddd, 1H, $J_{3e/3a}$ = 13 Hz, $J_{3e/2a}$ = 5 Hz, $J_{3e/2e}$ = 2.5 Hz, 3-He), 1.84 (tdd, 1H, $J_{2a/2e}$ = $J_{2a/3e}$ = 5 Hz, $J_{2a/7}$ = 1 Hz, 2-Ha), 1.90 (dddd, 1H, $J_{5e/5a}$ = 12 Hz, $J_{5e/6a}$ = 4 Hz, $J_{5e/6e}$ = 3,5 Hz, $J_{5e/4a}$ = 3 Hz, 5-He), 2.13 (dddd, 1H, $J_{6a/6e}$ = 13 Hz, $J_{6a/5a}$ = 12.5 Hz, $J_{6a/5e}$= 4 Hz, $J_{6a/7}$ = 1 Hz, 6-Ha), 2.28 (dddd, 1H, $J_{6e/6a}$ = 13 Hz, $J_{6e/5a}$ = 4 Hz, $J_{6e/5e}$ = 3.5 Hz, $J_{6e/2e}$ = 2.5 Hz, 6-He), 2.66 (s, 3H, N-$CH_3$), 3.44 (dq, 1H, $J_{2e/3a}$ = $J_{2e/3e}$ = $J_{2e/6e}$ = 2.5 Hz, 2-He), 6.40 (s, breit, 1H, 7-H), 7.48-7.66 (m, 3H, Ar-H), 7.84-7.96 (m, 2H, Ar-H).

$C_{18}H_{27}NOS$ (305.5) Ber. C 70.77 H 8.91 N 4.58
Gef. C 70.43 H 9.17 N 4.40

<u>$^1$H-NMR</u> (300 MHz, $CDCl_3$): δ = 0.82 (s, 9H, tert-Butyl-H), 1.07-1-32, (m, 3H, 3-Ha, 3-He, 5-Ha), 1.56 (tdd, 1H, $J_{6a/6e}$ = 13 Hz, $J_{6a/5a}$ = 13 Hz, $J_{6a/5e}$ = 3 Hz, $J_{6a/7}$ = 1 Hz, 6-Ha), 1.82-1.98 (m, 2H, 4-Ha, 5-He), 2.07 (m, 1H, $J_{2a/2e}$ = 14 Hz, $J_{2a/3a}$ = 14 Hz, $J_{2a/7}$ = 1 Hz, $J_{2a/3e}$ = ? Hz, 2-Ha), 2.29(dddd, 1H, $J_{2e/2a}$ =14 Hz, $J_{2e/3a}$ = 2.5 Hz, $J_{2e/6e}$ = 2 Hz, 2-He), 2.65 (s. 3H, N-$CH_3$),3.54 (dq, 1H, $J_{6e/6a}$ = 13 Hz, $J_{6e/2e}$ = $J_{6e/5a}$ = $J_{6e/5e}$ = 2 Hz, 6-He),6.40 (s, 1H, breit, 7-H), 7.46-7.54 (m, 3H, Ar-H), 7.80-7.96 (m, 2H, Ar-H).

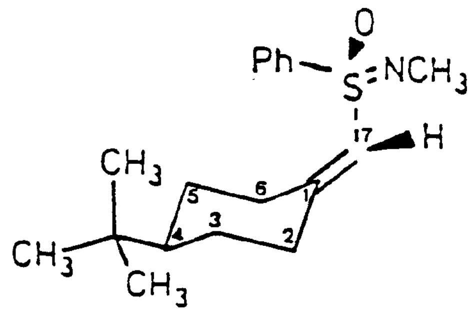

(-)-(aR)- 8b

$^{13}$C-NMR (75.47 MHz, $CDCl_3$): Gemisch aus 8a und 8b
8a: δ = 27.41 (q. $CMe_3$), 28.06, 28.67, 28.95 (3xt, 2- oder 6-C, 3-C), 29.12 (q, N-$CH_3$), 32.28 (s, $CME_3$), 37.26 (t, 2- oder 6-C), 47.25 (d, 4-C), 123.54 (d, 7-C), 128.59, 128.99 (2xd, Ar-c), 132.06 (d, Ar-C), 141.46 (s, Ar-C), 159.91 (s, 1-C).

Für 8b werden nur die C-Atome aufgelistet, die eine andere chemische Verschiebung als in 8a haben.
8b: δ = 27.51 (3xq, $CME_3$), 29.31 (q, N-$CH_3$), 32.43 (s, $CMe_3$), 37.34 (t, 2- oder 6-C), 47.38 (d, 4-c), 123.44 (d, 7-c), 128.54, 128.99 (2xd, Ar-c).
MS (EI, 70 eV): m/z(%) 305 ($M^+$, 6), 169 (14), 142 (21), 141 (24), 91 (22), 77 (25), 57 ($C_4H_9$, 100), 41 (80).

7.) [3aS-(2αβ, 3αβ, 4β, 5α, 6aβ)]-5-[[(1, 1-dimethyl)-dimethylsilyl]-oxy]-4-[[[1,1-dimethyl)-dimethylsilyl]-oxy]-methyl]-2β (R)-[[(N-methyl-S-phenyl)-sulfonimidoyl]-methyl]-hexahydro-pentalen]-2α(1H)ol (10)

152 mg (0.9 mmol) S-1 (ee ≧ 95 %) werden nach A 1 mit 360 mg (0.9 mmol) [3aS- (3aα, 4α, 5β, 6aα)]-5-[[(1,1-dimethylethyl)-dimethylsilyl]oxy]-4-[[[(1,1-dimethylethyl)-dimethylsilyl]-oxy]-methyl]-hexahydro-2 (1H)-pentalenon (9) umgesetzt. FC an 100 g Kieselgel (Hexan/Essigester 3:1) liefern 480 mg (94 %) 10 als farbloses Öl, das nach einigen Tagen im Kühlschrank durchkristallisiert. Die Kristallisation kann auch durch mehrmaliges Einengen mit wfr. Hexan beschleunigt werden, Schm. 65-66 °C, $[\alpha]_D 20$ = +13°, $[\alpha]^{20}_{546}$ = +24° (c = 0.24 in Aceton). Die Drehwerte sind sehr stark temperaturabhängig.

$C_{29}H_{53}NO_4S\ Si_2$ (567.99) Ber. C 61.33 H 9.41 N 2.47
Gef. C 61.52 H 9.51 N 2.39

$^1$H-NMR (300 MHz, $CDCl_3$): δ = 0.03, 0.04, 0.05, 0.06 (4xs, 12H, SitBuMe$_2$), 0.88, 0.90 (2xs, 18H, SitBuMe$_2$), 1.67 (ddd, 1H, $J_{6a/6\beta}$ = 14 Hz, $J_{6a/5\beta}$ = 9 Hz, $J_{6a/6a\beta}$ = 7-8 Hz, 6-Ha), 1.67 (dd, 1H, $J_{1\beta/1a}$ = 13,5 Hz, $J_{1\beta/6a\beta}$ = 8 Hz, 1-oder 3-Hß), 1.83 (dd, 1H, $J_{1a/1\beta}$ = 13.5 Hz, $J_{1a/6a\beta}$ = 4 Hz, 1-oder 3-Ha), 1.96-2.1 (ddt, $J_{4a/8}$ = 7,5 Hz, J = 4 Hz, 4-Ha und ddd, 1H, $J_{6\beta/6a}$ = 14 Hz, J = 7 Hz, 6-Hß), 2.15 (dd, 1H, $j_{3a}$ = 13 Hz, $J_{3a/3a\beta}$ = 2 Hz, 3-oder 1-Ha), 2.2-2.40 (dd, 1H, $J_{3a/3\beta}$ = 13 Hz, 3-oder 1-Hß und m, 1H, 3a-Hß und m, 1H, 6a-Hß), 2-62 (s; 3H, N-$CH_3$), 3.05, (d, 1H, J = 14 Hz, 7-H), 3.49 (d, 1H, J = 14 Hz, 7-H), 3,63 (2xdd, 2H, J = 13 Hz, $J_{8/4a}$ = 4 Hz, 8-H), 3.90 (m, 1H, J = 9 Hz, J = 7.5 Hz, 5-Hß), 6.35 (s, breit, 1H, -OH), 7.52-7.68 (m, 3H,Ar-H), 7.84-7.92 (m, 2H, Ar-H).

$^{13}$C-NMR (75.47 MHz, $CDCl_3$): δ = -5.4, -5.3, -4.7, -4.4 (4xq, SitBuMe$_2$), 18, 1. 18.4 (2xs, SitBuMe$_2$), 25.9, 26.1 (2xq, SitBuMe$_2$), 28.96 (q, N-$CH_3$), 37.2, 41.9 (2xd, 3a-C, 6a-C), 41.8, 44.0, 47.4 (3xd, 1-C, 3-C, 6-C), 56.2 (d, 4-C), 62.4, 64.4 (2xt, 7-C, 8-C), 74.8 )d, 5-C) 82.1 (s, 2-C), 129.1, 129.5 (2xd, Ar-C), 132.9 (d, Ar-C), 139.5 (s,

Ar-C).
MS (EI. 70 eV): m/z (%) = 567 ($M^+$, 3), 552 ($M^+$-15.3) 510 ($M^+$-$C_4H_9$, 72) 341 (48), 156 (51), 147 (100), 131 (23), 125 (PhSO, 35), 107 (20), 106 (21), 89 (28), 84 (32), 75 (33), 73 (95).

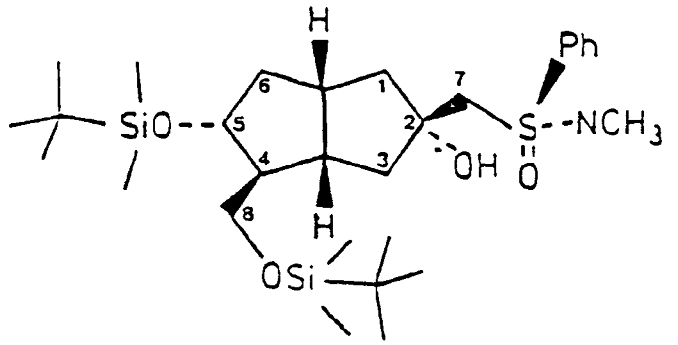

10

8.) [3aS-(2Z, 3aα, 4α, 5β, 6aα)]-S(R)-[5-[[(1,1-dimethylethyl)-dimethylsilyl]-oxy]-4-[[[(1,1-dimethylethyl)-dimethylsilyl]-oxy]-methyl]-hexahydro-2(1H)-pentalenoyliden]-methyl-N-methyl-S-phenyl-sulfoximin (11)

595 mg (1.05 mmol) 10 werden nach A 2 umgesetzt. 2 h nach Zugabe des zweiten Äquivalents n-Butyllithiumlösung wird aufgearbeitet. TC an 130 g Kieselgel (Hexan/Essigester 1:1) liefert neben 70 mg (12 %) unumgesetzten 10 490 mg (85 %, umsatzbezogen 96 %) 11 als farbloses Öl, das sehr langsam im Lauf mehrerer Tage im Kühlschrank kristallisiert, Schmp. 49-51°, $[\alpha]^{20}_D$ = -79°, $[\alpha]^{20}_{546}$ = -96° (c = 0.65 in aceton).
Nach dem $^1$H-NMR (300 MHz) enthält das diastereomerenreine Produkt noch zu 3-10 % die isomerisierte Verbindung 11, wobei noch nicht geklärt ist, ob die Isomerisierung basisch katalysiert vor der Aufarbeitung oder sauer katalysiert während oder nach der Aufarbeitung stattfindet.

$C_{29}H_{51}NO_3SSi_2$ (549.3) Ber. C 63.33 H 9.35 N 2.55
(549.3110, Gef. C 63.43 H 9.62 N 2.52
549.31117 Hochauflösung)

$^1$H-NMR (300 MHz, $CDCl_3$): δ = 0.0, 0.01, 0.03 (2xs, 6H und 1xs, 6H, SitBu$Me_2$), 0.84, 0.88 (2xs, 18H, Sit$Bu$Me$_2$), 1.27 (ddd, 1H, $J_{6ß/6a}$ = 13 Hz, $J_{6ß/5a}$ = 7.5 Hz, $J_{6ß/6aa}$ = 6 Hz, 6-Hß), 1.53 (ddt, 1H, $J_{4ß/5a}$ = 7.5 Hz, $J_{4ß/3aa}$ = 7.5 Hz, $J_{4ß/8}$ = 4 Hz, 4-Hß), 2.04 (ddd, 1H, $J_{6a/6ß}$ = 13 Hz, $J_{6a/5a}$ = 7,5 Hz, $J_{6a/6aa}$ = 7 Hz, 3-Ha), 2.34 (m, 2H, 3a-Ha, 6a-Ha), 2.39 (dd, 1H, J = 17-18 Hz, 1-Hß), 2.46 (dd, 1H, $J_{3a/3ß}$ = 18 Hz, $J_{3a/3aa}$ = 7 Hz, 3-Ha), 2.61 (dd, 1H, $J_{1a/1ß}$ = 17-18 Hz, $J_{1a/6aa}$ = 7.5 Hz, 1-Ha), 2.67 (s, 3H, N-$CH_3$), 2.95 (dd, 1H, $J_{3ß/3a}$ = 18 Hz, $J_{3ß/3aa}$ = 2 Hz, 3-Hß), 3.56 (m, 2H, 8-H), 3.90 (q, 1H, J = 7.5 Hz, 5-Ha), 6.24 (m, 1H, 7-H), 7.42 -7.60 (m, 3H, Ar-H), 7.8-7.9 (m, 2H, Ar-H).

Die Zuordnung der Protonen gelang durch Entkopplungsexperimente. Die eindeutige Festlegung der Z-Stereochemie der Doppelbindung erfolgte durch $^1$H-NOE Differenzmessung, durchgeführt von H. Günther, Siegen. Einstrahlung bei 7-H führte zu NOE für 1-Hα, 1-Hβ und beweist somit, daß der Tieffeldshift von 3-Hβ erwartungsgemäß durch den Anisotropieeffekt der Sulfoximinfunktion verursacht wird, also 3-Hβ und die Gruppierung cis-ständig angeordnet sind. Einstrahlung bei 3-Hβ führt zu einem NOE für 3-Hα und 4-Hβ, verknüpft also die Stereochemie der zwei Ringe eindeutig.
$^{13}$C-NMR (75.47 MHz, $CDCl_3$): δ = -5,4. -4.8, -4.5 (3xq, SitBu$Me_2$), 17.9, 18.3 (2 x s, Sit$Bu$Me$_2$), 25.8, 26.0 (2

x q, SitBume$_2$), 29.3 (q, N-$CH_3$), 35.2, 41.3 42.7 (3 x t, 1-C, 3-C, 6-C), 37.7, 42.9 (2 x d, 3a-C, 6a-C), 56.4 (d, 4-C), 61.9 (t, 8-C), 74.4 (d, 5-C), 122.2 (d, 7-C), 128.6, 129.0 (2 x d, Ar-C), 132.1 (d, Ar-C), 132.1 (d, Ar-C), 141.0 (s, Ar-C), 164.2 (s, 2-C).
MS (EI, 70 eV): m/z (%) = 549 ($M^+$, 48), 492 ($M^+$-$C_4H_9$, 100), 246 (33), 147 (33), 131 (28), 89 (18), 75 (26), 73 (100).

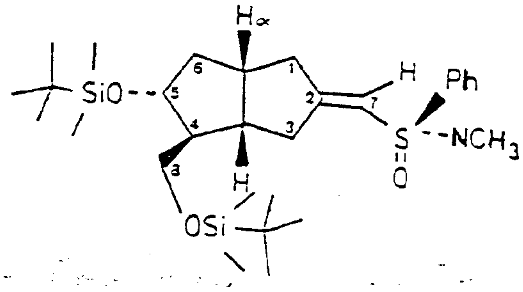

11

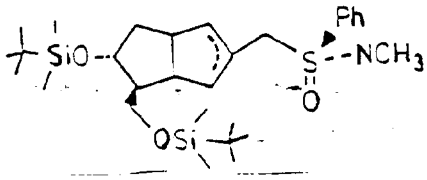

11 i

9.) (3aS- (2αβ, 3aβ, 4β, 5α, 6aβ)]-5-[[(1,1-dimethylethyl)-dimethylsilyl]-oxy]-4-[[[(1,1-dimethylethyl)-dimethylsilyl]-oxy]-methyl]-2β-(S)-[[N-methyl-S-phenyl)sulfonimidoyl]-methyl]-hexahydropentalen]-2α (1H)-ol (12)

305 mg (1.8 mmol) R-1 (ee ≧ 95 %) werden nach A 1 in 20 ml absoluten THF mit 715 mg (1.8 mmol) 9 umgesetzt. FC an 130 g Kieselgel (Hexan/Essigester 3:1) liefert 960 mg (94 %) 12 als farbloses Öl, das nur langsam im Kühlschrank kristallisiert, Schmp. 67°-68 °C, $[\alpha]_D^{20}$ = -42°, $[\alpha]^{20}_{546}$ = 50° (c = 1 in Aceton); 10 und 12 sind dünnschichtchromatographisch unterscheidbar durch mehrmalges Entwickeln mit Hexan/Essigester 6:1, 10 ist polarer als 12.

| | | | | |
|---|---|---|---|---|
| $C_{29}H_{53}NO_4SSi_2$ | Ber. | C 61.33 | H 9.41 | N 2.47 |
| | | C 61.55 | H 9.72 | N 2.39 |
| (567.99) | | | | |

$^1$H-NMR (300 MHz, $CDCl_3$): δ = 0.0, 0.01, 0.02 (2 x s, 6H und s, 6H, SitBuMe$_2$), 0.85, 0.87 (2x s, 18H, SitBuMe$_2$), 1.68 (dd, 1H, $J_{1ß/1a}$ = 13 Hz, $J_{1ß/6aß}$ = 8 Hz, 1- oder 3-Hß und ddd, 1H, $J_{6a/6ß}$ = 13 Hz, $J_{6/6aß}$ = 8 Hz, $J_{6/5ß}$ = 5.5 Hz, 6-H), 1.88 (dd, 1H, $J_{1a/1ß}$ = 13 Hz, $J_{1a/6aß}$ = 5 Hz, 1- oder 3-$H_a$), 1.98 (m, 1H, 4-H), 1.96-2.14 (dd, 1H, $J_{3ß/3a}$ = 13 Hz, $J_{3ß/3aß}$ = 7 Hz, 3-oder 1-Hß), 2.0-2.22 (2 x m, 2H, 6-H, 3a-Hß), 2.34-2.50 (m, 1H, 6a-Hß und dd, 1H, 3-oder 1-Ha), 2.60 (s, 3H, N-$CH_3$), 3.06 (d, 1H, J = 14 Hz, 7-H), 3.43 (d, 1H, J = 14 Hz, 7-H), 3.50 (dd, 1H, J = 11 Hz, $J_{8/4a}$ = 5.5 Hz, 8-H), 3.62 (dd, 1H, J = 11 Hz, $J_{8/4a}$ = 4 Hz, 8-H), 3.83 (dt, 1H, $J_{5ß/4a}$ = 7 Hz, $J_{5ß/6}$ = 5.5 Hz, 5-Hß), 6.42 (s, breit, 1H,-OH), 7.50-7.66 (m, 3H, Ar-H), 7.80-7.92 (m, 2H, Ar-H).

$^{13}$C-NMR (75.47 MHz, $CDCl_3$): δ = -5.6, -5.3, -4.7, -4.3 (4 x q, SitBuMe$_2$), 18.2, 18.5 (2 x s, SitBuMe$_2$), 18.2, 18.5 (2 x s, SitBuMe$_2$), 26.6, 26.1 (2 x q, SitBuMe$_2$), 28.9 (q, N-$CH_3$), 38.4, 41.4 (2 x d, 3a-C, 6a-C), 41.9, 45.4, 46.9 (3 x t, 1-C, 3-C, 6-C), 56.7 (d, 4-C), 63.4, 64.6 (2 x 6, 7-C, 8-C), 75.5 (d, 5-C), 82.2 (s, 2-C), 129.1, 129.6

(2 x d, Ar-C), 132.9 (d, Ar-C), 140 (s, Ar-C).
MS (EI. 70 eV): m/z (%) = 567 ($M^+$, 2), 552 ($M^+$ -15.5), 510 ($M^+$-$C_4H_9$, 53), 341 (29), 223 (19), 156 (42), 147 (50), 125 (23), 73 (40), 69 (30), 56 (100), 41 (73).
MS (FD): m/z = 568 ($M^+$+1), 567 ($M^+$), 510 ($M^+$ -$C_4H_9$).

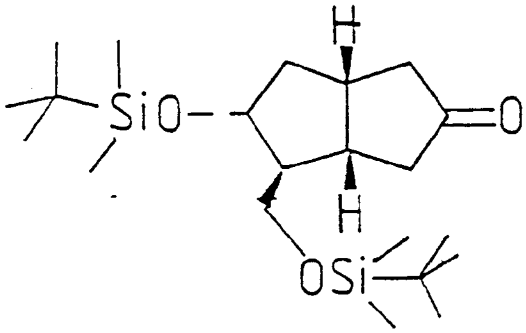

9

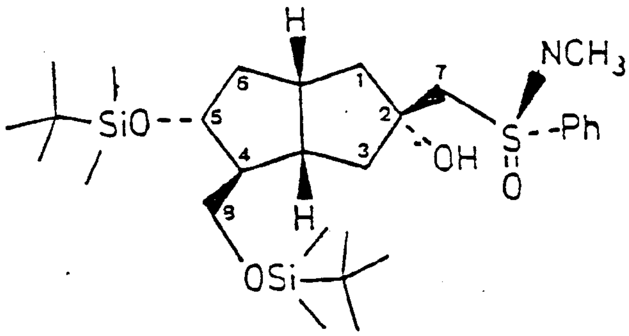

12

10.) [3aS-(2E, 3aα, 4α, 5β, 6aα)]-S(S)-[5-[[(1,1-dimethylethyl)-dimethylsilyl]-oxy]-4-[[[(1,1-dimethylethyl)-dimethylsilyl]-oxy]-methyl]-hexahydro-2 (1H)-pentalenoyliden]-methyl-N-methyl-S-phenylsulfoximin (13)

595 mg (1.05 mmol) 12 werden nach A 2 in 30 ml absoluten THF umgesetzt. FC an 100 g Kieselgel (Hexan/Essigester 1:1) liefert 525 mg (91 %) 13 als farbloses Öl, das im Kühlschrank kristalliert, Schmp. unterhalb Raumtemperatur, $[\alpha]^{20}_D$ = +59°, $[\alpha]^{20}_{546}$ = +71° (c = 0.39 in Aceton).

Im Gegensatz zu Beispiel 8 ist im $^1$H-NMR (300 MHz) von 13 keine Spur einer Isomerisierung zu erkennen. 11 und 13 sind ebenfalls dünnschichtchromatographisch unterscheidbar, wobei 11 polarer als 13 ist.

| | | |
|---|---|---|
| $C_{29}H_{51}NO_3SSi_2$ | Ber. | C 63.33 H 9.35 N 2.55 |
| | Gef. | C 63.04 H 9.44 N 2.34 |

$^{1}$H-NMR (300 MHz, $CDCl_3$): δ = 0.01, 0.02, 0.08 (1 x s, 6H, 2 x s, 6H, SitBu$Me_2$), 0.86, 0.87 (2 x s, 18H, SitBu$Me_2$), 1.26 (dt, 1H, $J_{6a/6ß}$ = 13 Hz, $J_{6/6aa}$ = $J_{6/5a}$ = 7.5 Hz, 6-H), 1.53 (ddt, 1H, $J_{4ß/5a}$ = $J_{4ß/3aa}$ = 7.5 Hz, $J_{4ß/8}$ = 6 Hz, $J_{4ß/8}$ = 4 Hz, 4-Hß), 2.08 (dt, 1H, $J_{6a/6ß}$ = 13 Hz, $J_{6/6aa}$ = $J_{6/5a}$ = 7.5 Hz, 6-H), 2.21 (dq, 1H, $J_{3aa/6aa}$ = $J_{3aa/4ß}$ = $J_{3aa/3a}$ = 7.5 Hz, $J_{3aa/3ß}$ = 3 Hz, 3a-Ha), 2.37 (m, 1H, $J_{6aa/3aa}$ = $J_{6aa/6a}$ = $J_{6aa/6ß}$ = 7.5 Hz, $J_{6aa/1^2}$ = 3 Hz, $J_{6aa/1ß}$ = 2 Hz, 6a-Ha), 2.24-2.65 (m, 3H, 1-Ha, 3-Ha, 3-Hß), 2.65 (s, 3H, N-$CH_3$), 2.87 (dd, 1H, $J_{1ß/1a}$ =18 Hz, $J_{1ß/6aa}$ = 3 Hz, 1-Hß), 3.50 (dd, 1H, J = 11 Hz, J = 6 Hz, 8-H), 3,59 (dd, 1H, J = 11 Hz, J = 4 Hz, 8-H), 3.83 (q, 1H, J = 7.5 Hz, 5-HA),6.24 (s, breit, 1H, 7-H), 7.42-7.56 (m, 3H, Ar-H), 7.80-7.90 (m, 2H, Ar-H).

Die Zuordnung der Doppelbindungskonfiguration gelingt eindeutig durch Entkopplungs- bzw. 2D-$^{1}$H-Messung. Durch Entkopplung bei 4-H läßt sich eindeutig der Brückenkopf 3a-H identifizieren. Wird nun bei 1-Hβ eingestrahlt, das aufgrund seines Tieffeldshifts bzgl. der Doppelbindung cis zur Sulfoximinfunktion angeordnet sein muß, vereinfacht sich nur das signal, das dem Brückenkopf 6a-Hα zugeordnet ist. Das signal 3a-Hα ändert sich nicht. Die 2D-Messung bestätigt die Zuordnung (durchgeführt von s. Braun, Darmstadt).

$^{13}$C-NMR (75.47 MHz, $CDCl_3$): δ = -5.5, -5.4, -4.9, -4.4 (4 x q, SitBu$Me_2$), 18.0, 18.3 (2 x s, SitBuMe$_2$), 25.8, 25,9 (2 x q, SitBuMe$_2$), 29.3 (q, N-$CH_3$), 36.5, 39.5, 41.3, 41.6, 41.7 (2 x d, 3 x t, 1-C, 3-C, 6-C, 3a-C, 6a-C), 55.8 (d, 4-C), 62.6 (t, 8-C), 74.4 (d, 5-C), 122.3 (d, 7-C), 128.6, 129.0 (2 x d, Ar-C). 132.1 (d, Ar-C), 141.0 (s, Ar-C), 164.1 (s, 2-C).

MS (EI, 70 eV): m/z (%) = 549 ($M^+$, 50), 534 ($M^+$-15.8), 492 ($M^+$-$C_4H_9$, 100), 147 (56), 131(23), 89(23), 73(100).

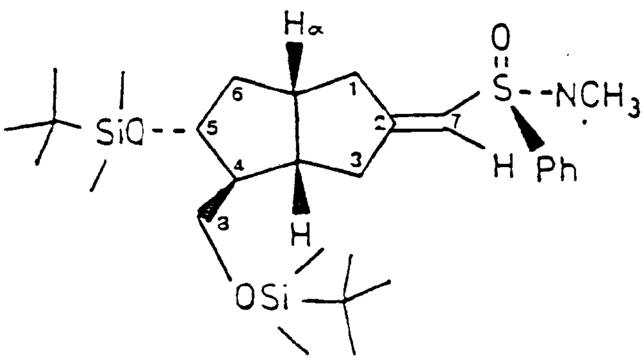

13

## Patentansprüche

1. Verfahren zur Herstellung von unsymmetrischen Olefinen der Formel I

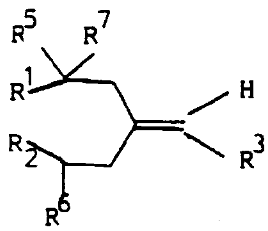

(I),

worin $R^1$ und $R^2$ eine gemeinsame Bindung oder den Rest

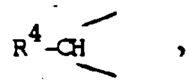

$R^3$ die Gruppe

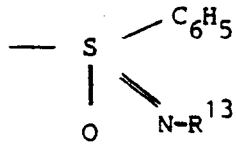

$R^4$ einen geradkettigen oder verzweigten Alkylrest mit 1-10 C-Atomen
$R^5$ und $R^6$ gleich oder verschieden sind und Wasserstoff, Alkyl mit 1-10 C-Atomen, Cycloalkyl mit 5-7 C-Atomen, Alkoxy mit 1-6 C-Atomen, Aryl mit 6-10 C-Atomen, Aralkyl mit 7-12 C-Atomen oder einen 5-7-gliedrigen Heterocycl us, der ein weiteres N-, O- oder S-Atom enthalten kann, oder wenn $R^1$ und $R^2$ eine gemeinsame Bindung darstellen $R^5$ und $R^6$ gemeinsam die Reste

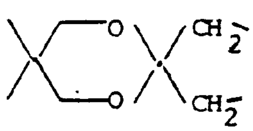 , 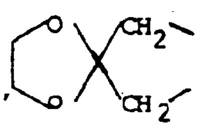 , 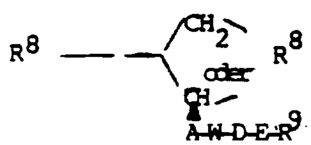 oder 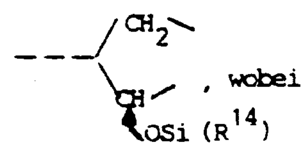 , wobei

$R^8$ Wasserstoff, Alkyl mit 1-10 C-Atomen oder $OR^{10}$ mit $R^{10}$ in der Bedeutung eines Wasserstoffs, eines Silylrestes oder eines Äther- oder Säurerestes darstellt,
A eine trans-CH=CH-Gruppe oder -C≡C-Gruppe,
W eine Hydroxymethylen- oder $-C(CH_3)(OH)$-Gruppe,
D eine Alkylengruppe mit 1-5 C-Atomen,
E eine -C≡C- oder $-CH = CR^{11}$-Gruppe,
$R^9$ eine Alkylgruppe mit 1-6 C-Atomen,
$DER^9$ Cycloalkyl mit 5-6 C-Atomen oder den Rest

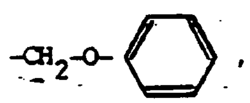 ,

$R^{11}$ Alkyl mit 1-4 C-Atomen,
$R^7$ die Reste $-(CH_2)_m-R^{12}$, oder

$$-(CH_2)_{m-o}-\left[Z_1-(CH_2)_{m-p}\right]_x-\left[Z_2-(CH_2)_{m-q}\right]_y R^{12},$$

Wasserstoff
$R^{13}$ Alkyl mit 1-4 C-Atomen oder einen Tosylrest,
$R^{14}$ Alkyl mit 1-4 C-Atomen, Phenyl oder Benzyl,
m = 2-20,
o, p und q≦16,
x, y = 0,1 oder 2,
$Z_1$ eine cis-CH=CH-Gruppe, eine trans-CH=CH-Gruppe oder eine -C≡C-Gruppe darstellt,
$Z_2$ Sauerstoff, Schwefel, eine NH-, eine N-Methylgruppe oder eine -C≡C-Gruppe bedeutet und

$R^{12}$ freies oder geschütztes Amino, Methylamino, Hydroxy, Carboxy, Mercapto oder Halogen bedeuten, dadurch gekennzeichnet, daß man an prochirale symmetrische Ketone der Formel II

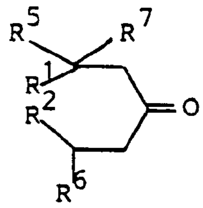 (II),

worin $R^1$, $R^2$, $R^5$, $R^6$ und $R^7$ die oben angegebenen Bedeutungen haben, das Lithiosulfoximin aus n-Butyllithium und einem N-substituierten S-Methyl-S-phenyl-sulfoximin-Derivat der Formel III

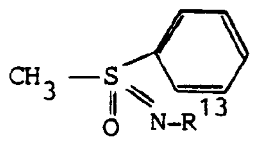 (III),

worin $R^{13}$ die bereits angegebene Bedeutung hat, anlagert und anschließend mit n-Butyllithium/Trimethylchlorsilan umsetzt.

## Claims

1. A process for the preparation of asymmetrical olefins of formula I

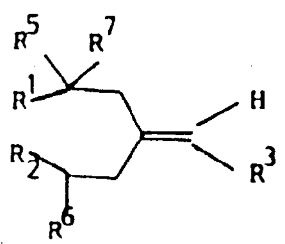 (I)

in which $R^1$ and $R^2$ represent a common bond or the radical

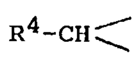 ,

$R^3$ represents the group

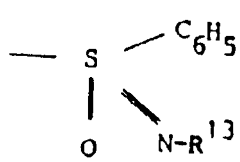

$R^4$ represents a straight-chain or branched alkyl radical having from 1 to 10 carbon atoms,
$R^5$ and $R^6$ are the same or different and each represents hydrogen, alkyl having from 1 to 10 carbon atoms, cycloalkyl having from 5 to 7 carbon atoms, alkoxy having from 1 to 6 carbon atoms, aryl having from 6 to 10 carbon atoms, aralkyl having from 7 to 12 carbon atoms or a 5-to 7-membered heterocycle that may contain an additional N, O or S atom, or, when $R^1$ and $R^2$ represent a common bond, $R^5$ and $R^6$ together represent the radical

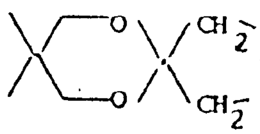, 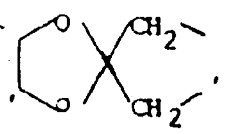, 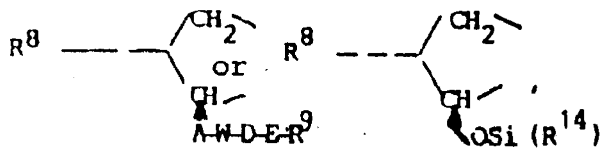

wherein $R^8$ represents hydrogen, alkyl having from 1 to 10 carbon atoms or $OR^{10}$ wherein $R^{10}$ represents a hydrogen atom, a silyl radical or an ether or acid radical,
A represents a trans-CH=CH- group or -C≡C- group,
W represents a hydroxymethylene or $-C(CH_3)(OH)-$ group,
D represents an alkylene group having from 1 to 5 carbon atoms,
E represents a -C≡C- or $-CH=CR^{11}-$ group,
$R^9$ represents an alkyl group having from 1 to 6 carbon atoms,
$DER^9$ represents cycloalkyl having 5 or 6 carbon atoms or the radical

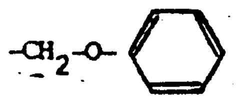

$R^{11}$ represents alkyl having from 1 to 4 carbon atoms,
$R^7$ represents the radical $-(CH_2)_m-R^{12}$, or

$$-(CH_2)_{m-o}-[Z_1-(CH_2)_{m-p}]_x-[Z_2-(CH_2)_{m-q}]_y-R^{12}$$

or hydrogen,
$R^{13}$ represents alkyl having from 1 to 4 carbon atoms or a tosyl radical,
$R^{14}$ represents alkyl having from 1 to 4 carbon atoms, phenyl or benzyl,
$\underline{m}$ = 2-20,
$\underline{o}$, $\underline{p}$ and $\underline{q} \leqq 16$,
$\underline{x}$, $\underline{y}$ = 0, 1 or 2,
$Z_1$ represents a cis-CH=CH- group, a trans-CH=CH- group or a -C≡C- group,
$Z_2$ represents oxygen, sulphur, an NH group, an N-methyl group or a -C≡C- group and
$R^{12}$ represents free or protected amino, methylamino, hydroxy, carboxy, mercapto or halogen, characterised in that there is added to prochiral symmetrical ketones of formula II

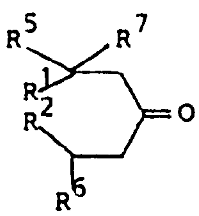

(II)

in which $R^1$, $R^2$, $R^5$, $R^6$ and $R^7$ have the meanings given above, the lithiosulphoximine of n-butyllithium and an N-substituted S-methyl-S-phenylsulphoximine derivative of formula III

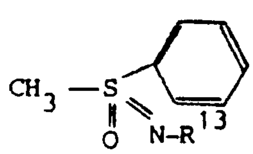

(III),

wherein $R^{13}$ has the meaning given above, and the product is then reacted with n-butyllithium/trimethylchlorosilane.

## Revendications

1. Procédé pour préparer des oléfines asymétriques, de formule I :

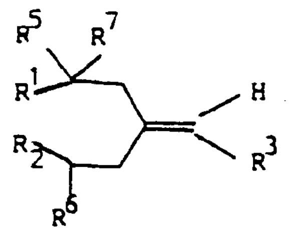

(I),

(dans laquelle $R^1$ et $R^2$ forment une liaison commune ou représentent le reste $R^4$ -CH ;
$R^3$ représente le groupe :

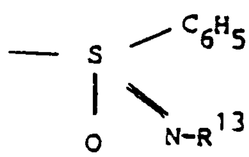

$R^4$ représente un reste alkyle linéaire ou ramifié ayant 1 à 10 atomes de carbone ;
$R^5$ et $R^6$ sont identiques ou différents et représentent chacun un atome d'hydrogène, un groupe alkyle ayant 1 à 10 atomes de carbone, cycloalkyle ayant 5 à 7 atomes de carbone, alcoxy ayant 1 à 6 atomes de carbone, aryle ayant 6 à 10 atomes de carbone, aralkyle ayant 7 à 12 atomes de carbone ou un hétérocycle comportant cinq à sept chaînons et qui peut contenir un autre atome de N, de O ou de S, ou bien, lorsque $R^1$ et $R^2$ représentent une liaison commune, $R^5$ et $R^6$ peuvent former ensemble les restes :

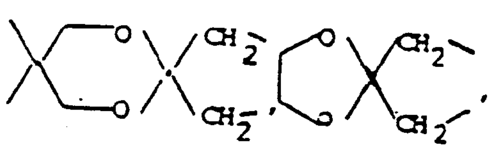

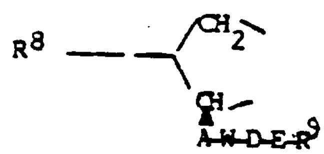

ou

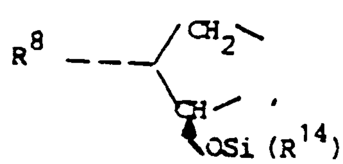

où
$R^8$ représente un atome d'hydrogène, un groupe alkyle ayant 1 à 10 atomes de carbone ou bien $OR^{10}$, le symbole $R^{10}$ représentant un atome d'hydrogène, un reste silyle ou un reste éther-oxyde ou acide,
A représente un groupe trans-CH=CH- ou -C≡C,
W représente un groupe hydroxyméthylène ou $-C(CH_3)(OH)$,
D représente un groupe alkylène ayant 1 à 5 atomes de carbone,
E représente un groupe -C≡C ou -CH = $CR^{11}$-,
$R^9$ représente un groupe alkyle ayant 1 à 6 atomes de carbone,
$DER^9$ représente un groupe cycloalkyle comportant 5 ou 6 atomes de carbone ou bien le reste :

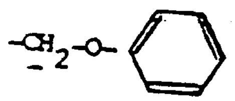 ,

$R^{11}$ représente un groupe alkyle ayant 1 à 4 atomes de carbone,
$R^7$ représente les restes $-(CH_2)_m-R^{12}$, ou $-(CH_2)_{m-o}-[Z_1-(CH_2)_{m-p}]_x-[Z_2-(CH_2)_{m-q}]_y-R^{12}$, ou un atome d'hydrogène,
$R^{13}$ représente un groupe alkyle ayant 1 à 4 atomes de carbone ou un reste tosyle,
$R^{14}$ représente un groupe alkyle ayant 1 à 4 atomes de carbone, un groupe phényle ou benzyle,
m vaut 2 à 20,
o, p et q sont égaux ou inférieurs à 16,
x, y valent 0, 1 ou 2,
$Z_1$ représente un groupe cis-CH=CH-, un groupe trans-CH=CH- ou un groupe -C≡C-,
$Z_2$ représente un atome d'oxygène ou de soufre, un groupe NH, un groupe N-méthyle ou un groupe -C≡C- et
$R^{12}$ représente un groupe amino, méthylamino, hydroxy, carboxy, mercapto libre ou protégé ou un atome d'halogène), procédé caractérisé en ce qu'on fixe, sur des cétones symétriques prochirales de formule II :

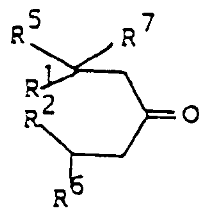 (II),

(dans laquelle $R^1$, $R^2$, $R^5$, $R^6$ et $R^7$ ont les sens indiqués ci-dessus) la lithiosulfoximine obtenue à partir de butyllithium et d'un dérivé, substitué sur l'azote, de S-méthyl-S-phényl-sulfoximine de formule III :

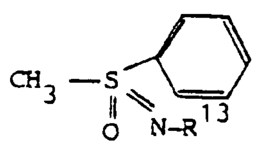 (III),

(dans laquelle $R^{13}$ a le sens déjà indiqué), et l'on fait ensuite réagir avec du butyllithium/triméthylchlorosilane.